# EUROPEAN PATENT APPLICATION

(11) **EP 4 478 087 A1**
(43) Date of publication of application: **18.12.2024**
(21) Application number: 23179118.7
(22) Date of filing: 14.06.2023
(51) Int. Cl.: G01S 13/04, G01S 13/56, G01S 13/88, G16H 40/20

(54) **A BED MONITORING SYSTEM AND METHOD**

(71) Applicant: Koninklijke Philips N.V., 5656 AG Eindhoven (NL)
(72) Inventor: KLABUNDE, Karin, 5656AG Eindhoven (NL); CLOUT, Ramon Antoine Wiro, 5656AG Eindhoven (NL); TOLHUIZEN, Ludovicus Marinus Gerardus Maria, 5656AG Eindhoven (NL); CHATTERJEA, Supriyo, 5656AG Eindhoven (NL)
(74) Representative: Philips Intellectual Property & Standards

(57) **Abstract**

A bed monitoring system comprises a radar system for mounting in a space where one or more beds may be located. The radar signals are used to identify the presence or absence of a bed at a set of one or more predefined bed locations within the space. This enables automatic detection of the presence of beds in predefined bed locations, without needing tags or communications systems. In a refinement of the system the same radar system is able, for an identified present bed, to identify the presence or absence of an occupant in the bed. The system then uses radar to monitor both the presence and occupancy of beds, with a single sensing system.

## Description

### FIELD OF THE INVENTION

This invention relates to bed monitoring systems, for monitoring the availability of beds, for example in a hospital environment.

### BACKGROUND OF THE INVENTION

As hospitals and their workflows become increasingly large and complex, it is not only the clinical procedures that require improvement, but hospital operations as well. One crucial workflow that is present in most hospitals and is yet to be addressed properly is that of bed management. There is a need for hospitals to understand if a bed slot is empty, occupied by a bed, or occupied by a patient and a bed. This information is used to decide which bed to assign a patient to when the patient is admitted to the hospital. The information is also used to decide when a bed needs to be cleaned, disinfected or transported.

Bed and bed slot status management is currently mostly a completely manual process in hospitals around the world. In order to find out the status of a bed, staff members usually need to make phone calls or manually search a particular bed in person and observe the status after it has been found. In addition, after finding a bed, the staff member needs to enter the status manually in the Electronical Medical Record (EMR). There are several problems with these manual approaches:
(i) It takes a lot of time and effort to find and finally allocate a bed. For example, patients can be waiting in the Emergency Department (ED) for up to several hours before they are admitted to the hospital due to bed allocation delays.
(ii) It is not uncommon for staff members to forget to enter data immediately indicating the change in bed status simply because they are busy with other activities. Such inefficiencies lead to longer length of stay for patients and to hospital overcrowding.

Bed management refers to the tracking of bed status, bed slot availability and location, as well as patient transfers. Patient transfers are often bottlenecks in many workflows. This step happens when a patient must be transferred to another department. If a patient is not quickly transferred, they are taking up precious space and time that another patient likely needs, especially in a high-intensity, high-stakes scenario such as the ED.

It is thus a challenge to provide clear information about the location and/or status of a bed. Because the patient always takes priority, it is common for assets in hospitals to be misplaced or left in a room where they are not supposed to be stored. This is a problem for hospital assets in general, but it is particularly impactful for hospital beds since hospitals do not overstock beds as much as other assets.

When a patient requires transferring to a new department, the receiving department needs to have a bed available for them. If information about beds is not constantly collected and updated, this process often means a manual search for an available bed. The increased lead time from this approach reduces the quality of patient care.

Another difficulty with bed management is the sourcing of a free bed slot (i.e., the intended location for a bed, rather than the bed itself). Even if a bed is available, once the patient is in the bed, they need to be placed in a bed slot. This can be an entire room, or a section of a room. With a lack of information, a similar process happens as in the procuring of a bed. The nurse is required to ask around, or manually search for a spot to place the patient and bed.

There are known attempts to automate or semi-automate the process. For example, there are known Real-Time Locating System (RTLS) solutions that are based on technologies such as infrared or Bluetooth. In these solutions, there is a need to tag both patients and beds, and to install additional infrastructure. Deploying a bed-level resolution RTLS on an enterprise-wide basis is extremely expensive.

There is therefore a need for a low cost and simple solution for bed management.

### SUMMARY OF THE INVENTION

The invention is defined by the claims.

According to examples in accordance with an aspect of the invention, there is provided a bed monitoring system, comprising:
a radar system for mounting in a space where one or more beds may be located, the radar system generating radar signals;
a processing system for receiving the radar signals, wherein the processing system is configured, from the radar signals, to:
   identify the presence or absence of a bed at a set of one or more predefined bed locations within the space.

This system uses radar to monitor the presence of beds using analysis of radar signals. The invention thereby provides a solution which avoids the need for manual inspection and does not require tags for the beds. Input from nursing staff is not actively required for determining available bed slots once the process of a patient transfer is initiated. After a patient transfer is requested, the system may be used to enable a bed location to be selected automatically or it may be used to provide available bed location options for a user to choose. For example, a bed-patient combination can be allocated to a free predefined bed location.

In a refinement described below, the system can also determine if a bed is occupied, so if a bed is in a predefined bed location but without a patient, the patient can be assigned to that bed.

In one approach, the assignment of a bed-patient combination to a bed slot or the assignment of a patient to an available bed can be implemented as an automatic process that the nursing staff are only tasked with executing, not also arranging. In another approach, the system can present to hospital staff the available bed slot (or unoccupied beds) to choose from, for example as a list or as colors on a floorplan.

The use of radar technology allows bed status detection with just a single radar per area where beds are to be placed (i.e., where there are predefined bed slots); no tags for beds are needed. Alternatively, one radar per bed could be used.

The radar system for example comprises a respective radar unit for each bed or for a subset of the beds in the space. A radar unit for each bed is easier to calibrate but requires more hardware. Each radar unit may comprise multiple antennas, for example for bed detection in different directions from the radar unit.

Each radar unit for example comprises a single input single output radar. This provides a low-cost implementation.

When multiple radar units share a space, the radar units are for example operated in time multiplex or frequency multiplex manner. A multiple antenna radar unit may distinguish between different areas based on the angular fields of view of the different antennas. However, a multiple antenna radar unit may also operate the different antennas in time multiplex or frequency multiplex manner.

The radar system for example comprises a radar unit that can distinguish between multiple beds in a single space based on range and/or angle from the radar unit. By choosing the location of the radar unit, different beds can be distinguished based on their distance to the radar unit and/or angular orientation relative to the radar unit. Angle separation is for example possible using multiple directional transmit and receive antennas, in known manner.

As mentioned above, for an identified present bed, the processing system may be further configured to identify the presence or absence of an occupant in the bed. Thus, a single radar system may be used for both bed detection (in the predefined bed slots) and occupant detection.

The presence or absence of a bed is for example derived from a range and/or angular profile whereas the presence or absence of an occupant is derived from a change in range profile over time.

This change in range profile corresponds to patient movement, and it for example represents a breathing pattern. The change in range profile is for example monitored over an observation window in the range of 5 seconds to 60 seconds, e.g., 5 seconds to 30 seconds. This provides a time window for sufficient patient movement to be captured.

The invention also provides a hospital bed management system comprising the bed monitoring system defined above and a system for allocating patients to unoccupied beds. The allocating system can also allocate beds to an available predefined bed location (i.e., bed slot). This allocation may be automatic or it may be performed after receiving user input, such as a nurse selection after a list of possible bed locations (or beds) has been presented to the nurse by the system.

The invention also provides a bed monitoring method, comprising:
receiving radar signals from a radar system mounted in a space where one or more beds may be located, the radar system generating radar signals; and
from the radar signals:
   identifying the presence or absence of a bed at a set of one or more predefined bed locations within the space.

This method uses radar to monitor the presence of beds, with a radar sensing system.

The method may comprise operating multiple radar units of the radar system in time or frequency multiplex manner. Thus, multiple radar units may operate in a shared space so that individual radar units need to be able to monitor a smaller number of bed locations.

The method may comprise distinguishing between multiple beds in a single space based on range and/or angle from the radar unit.

The method may comprise, from the radar signals and for an identified present bed, identifying the presence or absence of an occupant in the bed. Thus, the radar system may be used to determine both bed presence and bed occupancy.

The method may for example comprise deriving the presence or absence of a bed from a range and/or angular profile and deriving the presence or absence of an occupant from a change in range profile over time. This change in range profile corresponds to patient movement, for example representing a breathing pattern.

The invention also provides a computer program comprising computer program code which is adapted, when said program is run on a computer, to implement the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a bed monitoring system;
Fig. 2 shows a plot of magnitude versus range for one radar, with a bed location at a range of 1.5 to 2.5m;
Fig. 3 shows a superposition of range profiles sampled at different moments in time;
Fig. 4 shows a Fourier transform along the multiple frames to show the difference between an empty bed and a bed with a person; and
Fig. 5 shows a bed monitoring method.

### DETAILED DESCRIPTION OF EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a bed monitoring system, comprising a radar system for mounting in a space where one or more beds may be located. The radar signals are used to identify the presence or absence of a bed at a set of one or more predefined bed locations within the space. This enables automatic detection of the presence of beds in predefined bed locations, without needing tags or communications systems. In a refinement of the system, the same radar system is able, for an identified present bed, to identify the presence of absence of an occupant in the bed. The system then uses radar to monitor both the presence and occupancy of beds, with a single sensing system.

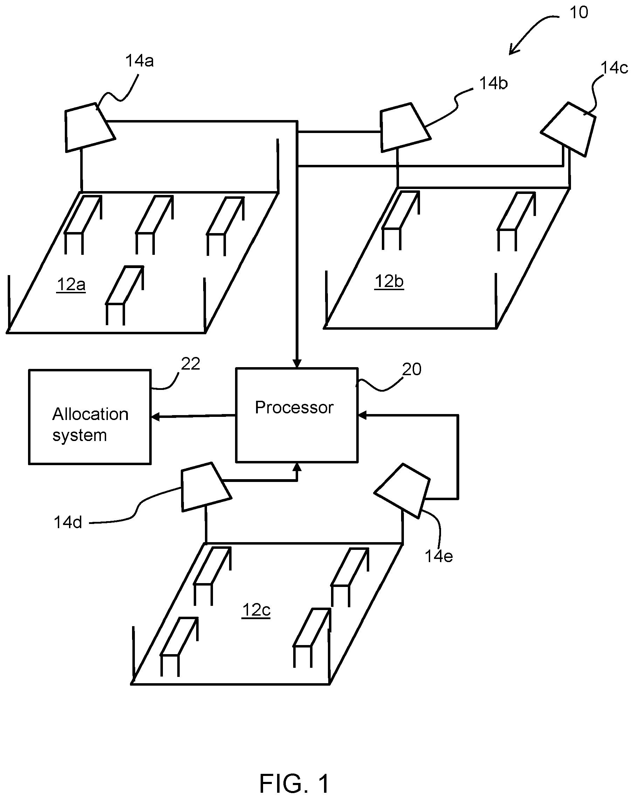

Fig. 1 shows a bed monitoring system 10 and shows three spaces 12a, 12b, 12c where one or more beds may be located. In each space, there is a set of predefined bed locations, and none, some, or all of the predefined bed locations may be occupied by an empty bed or a bed and patient at any given time.

Each space has one or more radar units 14a, 14b, 14c, 14d, 14e generating radar signals. In the example shown, there is one radar unit 14a for space 12a with multiple bed locations, two radar units 14b, 14c for space 12b with two bed locations, and two radar units 14d, 14e for the space 12c with more than two bed locations. Thus, in the space 12a, one radar unit monitors all of the multiple bed locations in that space, in the space 12b there is one radar unit for each bed location, and in the space 12c the radar units are each for monitoring a subset of the beds (so there are multiple radar units, at least one of which monitors multiple bed locations). In the cases where multiple radar units share a space, collision between the multiple radar units may be avoided by a time or frequency multiplex approach (i.e., operating the radar units at different times or frequencies). In combination, the various radar units constitute a radar system.

A radar unit may also have multiple antennas (so effectively functioning to provide separate range maps). The fields of view may be angularly separate, However, the different antennas may also operate a time or frequency multiplex approach.

A processing system 20 receives the radar signals and uses them to identify the presence or absence of a bed at a set of predefined bed locations within the corresponding space (or portion of the space).

In an optional refinement, for an identified present bed, the presence or absence of an occupant in the bed is also determined (sitting or lying).

Thus, the system then enables beds (or bed-patient combinations) to be allocated to empty bed slots, and it enables patients to be allocated to empty beds.

The radar system basically determines the presence of a bed, and optionally the presence of an occupant, based on a measured range (i.e., depth) profile, relative to calibration information. The range profile may also have angular resolution. The calibration information is for example the shape characteristics of the room with no bed present, and the shape characteristics of the room with beds present but no occupants.

This solution avoids the need for manual inspection and does not require tags for beds, or in the refinement of the system, for patients. Patient transfer can be performed with minimum manual intervention. A free bed can be selected automatically, a patient can be assigned to that free bed and the bed-patient combination can also be allocated automatically to a free bed slot, or assistance can be provided to hospital staff for them to make the allocation. For example, the hospital staff may be presented with a list of options to choose from.

Each radar unit comprises a radar sensor which may be placed at the ceiling of each monitored space, which is typically a hospital room.

For a bed occupant, the radar unit can also detect a respiration rate and heart rate in known manner. Thus, the processing system may be further configured to derive, from the radar signals, vital signs information for a patient in an occupied bed. The information gathered from the radar system can be used to automatically update status in EMR and other related applications.

The information collected by the processor 20 is then provided to a bed allocation system 22 which enables automated patient bed allocation or enables a simpler manual bed allocation for the user.

The initial installation of the radar system is followed by a calibration procedure. This involves collecting room characteristics with the empty room as well as assignment of locations to beds.

For each predefined bed location, the radar system is able to detect an empty bed slot or a bed slot with a bed. In the refined version, the system is also able to distinguish between a bed slot with an empty bed or a bed slot with an occupied bed. If the system is designed with one radar unit per bed, as mentioned above, the same calibration is used for capturing empty room characteristics but the set-up of the system becomes easier since each radar unit monitors only one potential bed location.

When a radar unit covers multiple beds, a sufficient resolution is required to distinguish the beds from each other. If the bed-to-radar distance is different for each bed, separation between the beds is possible based on range. For this purpose, a sufficiently high range resolution is required such as 5 cm to 10 cm, and there are then also constraints on the room configuration. The radar units of the radar system may for example be placed in such a way that each bed is visible in a separate range (bin).

If the bed-to-radar distance is similar for multiple beds, separation in angle is possible. This requires a radar unit with multiple antennas to provide a large field of view. For example, a 60GHz radar with 3 transmitters and 4 receivers has been tested and found able to distinguish between several beds as long as there were no beds at the same angular distance and the same range. A radar with a greater number of transmit antennas and receive antennas may be used to enable also more challenging configurations.

Calibration is needed to calibrate the radar in its environment. This is a standard procedure using corner reflectors. The calibration is performed with the radar system in its deployment location in a first phase without beds and in a second phase with beds. From this second calibration, the position of each bed in its bed slot can be determined in terms of angular separation and associated range bins.

Once the system is installed and calibrated, from the range profile and angular position, it can be computed if a bed slot is occupied. In particular, a bed can be detected by comparing the measured range profile with the empty room profile (from the calibration).

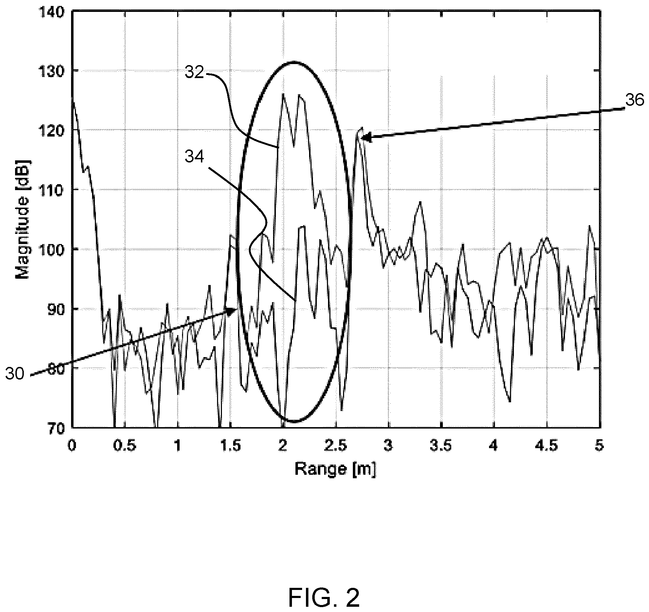

Fig. 2 shows a plot of magnitude versus range for one radar, with a bed location at a range of 1.5 to 2.5m shown in the ring 30.

Plot 32 is with a bed present and plot 34 is without a bed present. Region 36 shows a strong reflection from the room (which does not change depending on the presence of the bed).

By measuring changes in the range profile at the location where a person is expected on a bed, a breathing pattern can also be deduced and hence, the presence of a person on a bed.

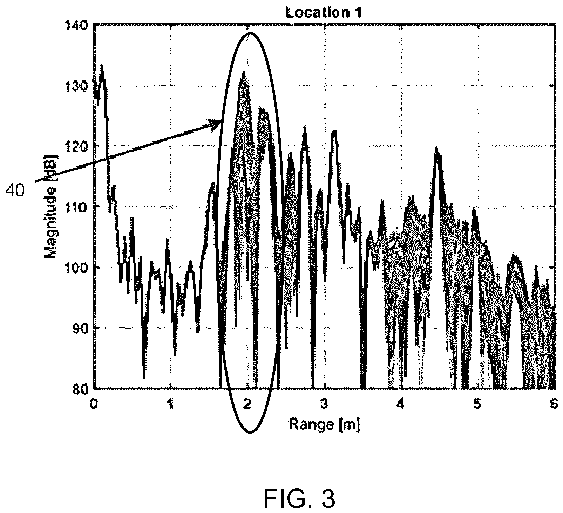

Fig. 3 shows a superposition of range profiles (sampled at different moments in time). In this example, the variation of the profiles is especially very large around the range bin 40 where a bed is expected (in this case around 2m). A sufficiently large observation window (from few seconds up to 30 seconds) is needed to distinguish changes in range profile due to the presence of a person from noise.

The method above may be used for a single bed, in which case only a single antenna pair is used for transmitting and receiving, or for multiple beds whereby multiple beds can be distinguished either by radar placement (placing the radar such a way that each bed appears in a separate range bin), or by having multiple antennas to provide information in the angular domain. The same approach may also be used to identify and distinguish multiple occupied beds in a single room.

When a radar is used per bed, a single input single output (SISO) radar can be used with very directive antennas and with low bandwidth. If there are multiple beds in a room, each radar can be operated in a different time slot or on a different frequency band.

Observing a person on a bed can be done by analysis of the range-doppler map with a long observation window overall (26 seconds) combined with Fourier analysis.

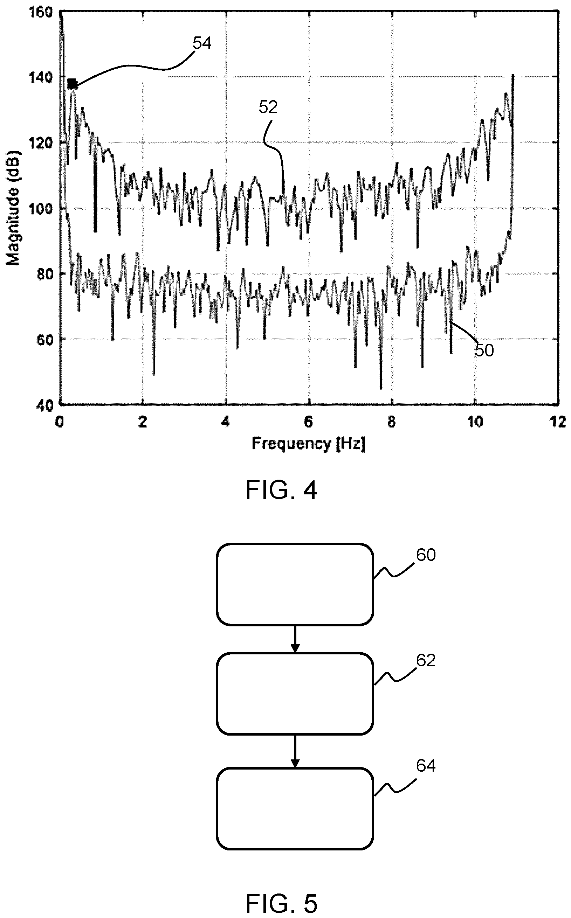

As shown in Fig. 4, a Fourier transform along the frames then shows a clear difference between an empty bed (plot 50) and a bed with a person (plot 52). An additional peak 54 around 0.3Hz in plot 52 is associated with the breathing pattern of a person on the bed.

Fig. 5 shows a bed monitoring method, comprising:
in step 60 receiving radar signals from a radar system mounted in a space where one or more beds may be located, the radar system generating radar signals.

In step 62, the presence or absence of a bed at a set of one or more predefined bed locations within the space is identified. Optionally, in step 64, for an identified present bed, the presence of absence of an occupant in the bed is determined.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A bed monitoring system, comprising:
a radar system (10) for mounting in a space where one or more beds may be located, the radar system generating radar signals; and
a processing system (20) for receiving the radar signals, wherein the processing system is configured, from the radar signals, to:
identify the presence or absence of a bed at a set of one or more predefined bed locations within the space.

2. The bed monitoring system of claim 1, wherein the radar system comprises a respective radar unit for each predefined bed location in the space.

3. The bed monitoring system of claim 1, wherein the radar system comprises a respective radar unit for a subset of the predefined bed locations in the space.

4. The bed monitoring system of claim 2 or 3, wherein each radar unit comprises a single input single output radar.

5. The bed monitoring system of claim 2, 3 or 4, wherein the radar units are operated in time or frequency multiplex manner.

6. The bed monitoring system of claim 1, wherein the radar system comprises a radar unit that can distinguish between multiple predefined bed locations in a single space based on range and/or angle from the radar unit.

7. The bed monitoring system of any one of claims 1 to 6, wherein for an identified present bed, the processing system is further configured to identify the presence or absence of an occupant in the bed.

8. The bed monitoring system of claim 7, wherein the presence or absence of a bed in predefined bed locations is derived from a range and/or angular profile and the presence or absence of an occupant is derived from a change in range profile over time.

9. A hospital bed management system comprising the bed monitoring system of any one of claims 1 to 8 and a system for allocating patients to unoccupied beds.

10. A bed monitoring method, comprising:
(60) receiving radar signals from a radar system mounted in a space where one or more beds may be located, the radar system generating radar signals; and
from the radar signals:
(62) identifying the presence or absence of a bed at a set of one or more predefined bed locations within the space.

11. The method of claim 10, comprising operating multiple radar units of the radar system in time or frequency multiplex manner.

12. The method of claim 10 or 11, comprising distinguishing between multiple predefined bed locations in a single space based on range and/or angle from radar units of the radar system.

13. The method of claim 10, 11 or 12, further comprising:
(64) for an identified present bed and from the radar signals, identifying the presence or absence of an occupant in the bed.

14. The method of claim 13, comprising deriving the presence or absence of a bed from a range and/or angular profile and deriving the presence or absence of an occupant from a change in range profile over time.

15. A computer program comprising computer program code which is adapted, when said program is run on a computer, to implement the method of any one of claims 10 to 14.
